(19) Europäisches Patentamt
European Patent Office
Office européen des brevets

(11) Numéro de publication: 0 091 330
A1

# (12) DEMANDE DE BREVET EUROPEEN

(21) Numéro de dépôt: 83400447.5

(22) Date de dépôt: 04.03.83

(51) Int. Cl.³: C 07 C 103/52

(30) Priorité: 07.04.82 FR 8206066

(43) Date de publication de la demande: 12.10.83 Bulletin 83/41

(84) Etats contractants désignés: AT DE GB NL SE

(71) Demandeur: LABORATOIRES HUMAN PHARM S.A.
94, rue Edouard Vaillant
F-92532 Levallois Perret Cedex(FR)

(72) Inventeur: Gourbault, Maurice Jean
10 rue Greuze
F-75016 Paris(FR)

(72) Inventeur: Chardin, Arlette
46 rue Gabriel Péri
F-92300 Levallois Perret(FR)

(72) Inventeur: Dressaire, Gilles
Appt D 1152 24 Avenue de Savigny
F-93600 Aulnay s/Bois(FR)

(74) Mandataire: Hirsch, Marc-Roger
34 rue de Bassano
F-75008 Paris(FR)

## (54) Procédé nouveau de production de l'aspartame.

(57) La présente invention a pour objet un procédé de production de l'aspartame par synthèse qui consiste à protéger la fonction amine libre de l'acide aspartique, à activer la fonction acide libre de l'acide aspartique protégé par production d'un anhydride interne protégé, à condenser cet anhydraide interne protégé avec l'ester méthylique de phénylalanine et à déprotéger la fonction amine du dérivé obtenu pour produire l'aspartame.

Ce procédé est caractérisé en ce que l'acide aspartique de départ est protégé par production d'une base de Schiff de l'acide aspartique. Elle permet d'obtenir un mélange d'isoméres d'aspartame enrichi en l'isomère α.

EP 0 091 330 A1

Croydon Printing Company Ltd.

PROCEDE NOUVEAU DE PRODUCTION
DE L'ASPARTAME

La présente invention a pour objet un procédé nouveau de production de l'aspartame sous la forme d'un mélange d'isomères comportant une proportion élevée de l'isomère α. Elle se rapporte également à l'aspartame ainsi produit.

L'aspartame, ester méthylique de L-aspartyl-L-phenylalanine de formule:

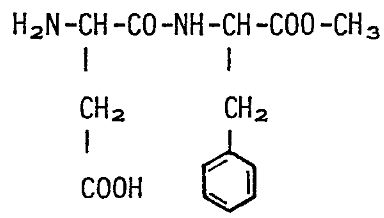

est un produit bien connu, notamment pour ses propriétés édulcorantes, qu'il est possible de préparer par diverses méthodes qui ont pratiquement toutes en commun les quatre étapes suivantes:

- une première étape dite de protection de la fonction amine libre de l'acide aspartique;
- une deuxième étape dite d'activation de la fonction acide libre de l'acide aspartique protégé, conduisant à la formation d'un anhydride interne protégé;
- une troisième étape dite de condensation de l'anhydride interne protégé avec l'ester méthylique de phenylalanine;
- une quatrième étape dite de déprotection de la fonction amine initialement protégée et d'obtention de l'aspartame.

En fait, ces méthodes de préparation diffèrent principalement par le choix du groupement protecteur employé, à savoir:

- l'emploi en tant que groupe protecteur du groupe carbobenzyloxy {article de Le Quesne et Young, "Synthesis of L-aspartyl peptides from carbobenzyloxy-L-aspartic anhydrides (J. Am. Chem. Soc. 1952 p. 24)};

- l'emploi en tant que groupe protecteur du radical formyle, du radical carbobenzyloxy et du radical para-methoxy-carbobenzoxy (BF 70 15787);
- l'emploi en tant que groupe protecteur du phosgène pour former le N-carboxyanhydride de l'acide L-aspartique (brevet Sud-Africain 67/02190 mis à la disposition du public le 18 octobre 1967).

Outre la méthode en quatre étapes précitées, on connait notamment une méthode consistant:

- à protéger sur l'acide aspartique la fonction amine par un groupe benzyloxycarbonyle et la fonction β-carboxy par un groupe ester benzylique;
- à transformer le groupe α-carboxy en fonction ester de p-nitrophenyle pour obtenir le N-benzyloxycarbonyl-L-aspartate d'α-p-nitrophenyle et β-benzyle (cf l'article de S. Guttmann dans la revue Helv. Chem. Acta, 44,721 (1961);
- à faire réagir cette substance avec l'ester méthylique de phénylalanine pour déplacer le groupe ester de p-nitrophényle et obtenir l'ester méthylique de la β-benzyl-N-benzyloxy-carbonyl-L-aspartyl-L-phénylalanine;
- à éliminer les groupes protecteurs N-benzyloxy-carbonyle et O-benzyle par hydrogenolyse.

Cette méthode est également décrite dans le brevet Sud-Africain n° 6702190 (cité infra).

Toutes ces méthodes conduisent à des rendements relativement satisfaisants en aspartame, mais ne sont pas très faciles à mettre en oeuvre. Par ailleurs elles conduisent à l'obtention de mélanges d'isomères dont le rapport des isomères α/β est de l'ordre de 1/1 à 2/1.

Par ailleurs, les méthodes faisant emploi de deux groupements protecteurs nécessitent de nombreuses matières premières et leur rendement est relativement faible.

En outre, ces méthodes connues nécessitent des opérations d'isolement intermédiaires (par exemple par filtration, dessication, concentration sous pression réduite intermédiaires).

La présente invention a pour objet un procédé perfectionné de production d'aspartame qui permet de pallier les inconvénients des procédés connus.

Ce procédé perfectionné permet effectivement l'obtention de l'aspartame à partir de l'acide aspartique protégé sans isolement intermédiaire, ce qui rend plus aisée et moins onéreuse l'obtention de l'aspartame, et élimine de nombreuses opérations de séparation fastidieuses. De plus, l'aspartame obtenu est de façon surprenante très enrichi en l'isomère α, le rapport des isomères α/β étant compris entre 3/1 et 5/1.

La présente invention a pour objet un procédé de production d'aspartame qui consiste à protéger la fonction amine libre de l'acide aspartique, à activer la fonction acide libre de l'acide aspartique protégé par production d'un anhydride interne protégé, à condenser cet anhydride interne protégé avec l'ester méthylique de phénylalanine et à déprotéger la fonction amine du dérivé obtenu pour produire l'aspartame, ce procédé consistant à protéger l'acide aspartique de départ par production d'une base de Schiff de l'acide aspartique.

Selon un mode de réalisation du procédé de l'invention, ladite base de Schiff est produite par traitement de l'acide aspartique, d'un de ses sels alcalins ou d'un sel de cet acide avec une amine tertiaire, avec un composé oxo-aromatique de formule:

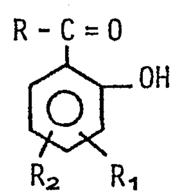

dans laquelle:

R est un atome d'hydrogène ou un radical alkyle inférieur, de préférence un radical méthyle;

$R_1$ et $R_2$, indépendamment l'un de l'autre consistent en un atome d'hydrogène ou d'halogène ou un groupe nitro ou encore, forment ensemble un noyau aromatique.

Ainsi, l'acide aspartique ou son sel alcalin ou d'amine, réagit avec ce composé oxo-aromatique selon le schéma réactionnel:

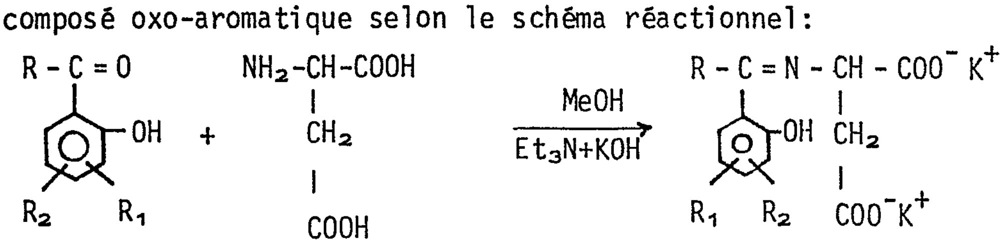


Selon un autre mode de réalisation du procédé de l'invention, la base de Schiff de l'acide aspartique est obtenue par mise en suspension dans un solvant alcoolique, par exemple le méthanol, de l'acide aspartique et du composé oxo-aromatique (A) précité, addition d'hydroxyde de potassium (KOH) et de triethylamine {$N-(CH_2CH_3)_3$} à la suspension ob-

tenue, addition d'acétate d'éthyle, cristallisation, filtration et séchage. La réaction entre l'acide aspartique et le composé oxo-aromatique est mise en oeuvre à une température comprise entre 0° et 65°C.

Selon une forme d'exécution de l'invention, la base de Schiff ainsi produite est mise en suspension dans un agent convenable et à la suspension ainsi obtenue sont ajoutés successivement;

- un agent déshydratant,
- après au moins une heure d'agitation, l'ester méthylique de phénylalanine, en solution dans l'agent de suspension précité,
- après au moins cinq heures d'agitation, de l'acide chlorhydrique,

le mélange biphasique obtenu est alors séparé et on neutralise la phase aqueuse par addition d'une solution alcaline. L'aspartame, lors de la neutralisation, se sépare par cristallisation.

De préférence, l'agent convenable pour la mise en suspension est le dichlorométhane ou l'acétate d'éthyle.

Selon une autre forme d'exécution de l'invention, l'agent déshydratant consiste en un anhydride d'acide gras ou un halogénure d'acide, tel par exemple le trichlorure de phosphore $PCl_3$.

A titre de composés oxo-aromatiques (A) susceptibles d'être employés dans l'invention on peut citer les composés suivants:

. hydroxy-2-naphtaldéhyde-1,
. hydroxy-2-acetophénone,
. hydroxy-2-benzaldéhyde,
. hydroxy-2-chloro-5-acétophénone,
. hydroxy-2-dichloro-3,5-acétophénone,
. hydroxy-2-chloro-5-benzaldéhyde,
. hydroxy-2-dichloro-3,5-benzaldéhyde,
. hydroxy-2-nitro-5-acétophénone,
. hydroxy-2-dinitro-3,5-acétophénone,
. hydroxy-2-nitro-5-benzaldéhyde,
. hydroxy-2-dinitro-3,5-benzaldéhyde

En fait, le procédé selon l'invention est surprenant non seulement parce qu'il permet l'obtention de l'aspartame avec un nombre extrêmement limité d'opérations d'isolement intermédiaire mais également parce qu'il est parfaitement inattendu qu'une molécule aussi labile que celle des bases de Schiff obtenues puisse être employée pour la protection de l'acide aspartique.

De plus, il est surprenant, et parfaitement inattendu que le mélange d'isoméres d'aspartame obtenu contienne une proportion relativement élevée

de l'isomère α, pouvant représenter 75 à 85% du mélange d'isomères obtenu, cet isomère α ayant seul les propriétés édulcorantes recherchées.

D'autres buts et avantages de l'invention apparaîtront à la lecture de la description suivante et des exemples donnés à titre non limitatifs.

Exemple 1

21,3 g d'acide aspartique (0,16 mole) et 27,6 g d'hydroxy-2-naphtaldéhyde-1 (0,16 mole) sont mis en suspension dans 96 ml de méthanol. 32,4 g de triéthylamine (0,32 mole) et 21,12 g d'hydroxyde de potassium (85%) dans 96 ml de méthanol sont ajoutés successivement.

Le mélange est porté à la température du reflux et maintenu à cette température pendant trois à quatre heures, puis refroidi. La base de Schiff produite précipite. Après filtration, sont obtenus 57,3 g (98,5% de la théorie) de cristaux. La base de Schiff est mise en suspension dans 400 ml d'acétate d'éthyle. 20 g d'acide acétique glacial et 10 g de trichlorure de phosphore sont alors successivement ajoutés sous agitation à 0° en 15 minutes.

L'agitation est continuée pendant une heure. 28,5 g de méthylester de L-phénylalanine (0,16 mole) en solution dans le solvant initialement choisi sont ajoutés et l'agitation est continuée 10 à 15 heures.

80 ml d'acide chlorhydrique 2 N sont ajoutés et l'agitation est continuée. Le mélange biphasique obtenu est séparé et la phase aqueuse est doucement neutralisée par addition de carbonate de sodium.
L'aspartame précipite lentement (point de fusion: 210°C -avec décomposition-)
Rendement 40 g (85% de la théorie).
Rapport isomère α/isomère β = 4/1. La formule du produit est: $C_{14}H_{18}N_2O_5$

L'analyse élémentaire du produit obtenu conduit aux résultats suivants:

| | | | |
|---|---|---|---|
| . observés: | C: 56,9% | H: 6,1% | N: 9,5% |
| . calculés: | C: 57,13% | H: 6,18% | N: 9,52% |

Exemple 2

53,2 g d'acide L-aspartique (0,4 mole) et 59,9 g d'hydroxy-2-acétophénone (0,44 mole) sont mis en suspension dans 240 ml de méthanol. 81 g de triéthylamine (0,8 mole) et 52,8 g d'hydroxyde de potassium (0,8 mole) en solution dans 240 ml de méthanol sont alors ajoutés. Le mélange est porté à la température du reflux et maintenu à cette température pendant 3 à 4 heures. Après repos pendant 12 heures, la base de Schiff précipite. Par filtration on isole 125,4 g de cristaux (95,5%) de la théorie). 81,5 g de cette base de Schiff sont mis en suspension dans 400 ml de dichlorométhane. Ensuite 30 g d'acide acétique glacial et 15,7 g de trichlorure de

phosphore sont ajoutés successivement sous agitation à 0°C.

Après une à trois heures d'agitation, 45 g de L-phénylalanine méthylester sont ajoutés; l'agitation est continuée pendant 10 à 14 heures. Ensuite, 115 ml d'acide chlorhydrique 2 N sont ajoutés. Le mélange biphasique est séparé et la phase aqueuse est lentement neutralisée par du carbonate de sodium. L'aspartame cristallise progressivement (point de fusion: 210°C (dec.) Rendement 43,8 g (65% de la théorie). La formule du produit est $C_{14}H_{18}N_2O_5$ et le rapport isomère α/isomère β = 3.8/1.

L'analyse élémentaire du produit obtenu conduit aux résultats suivants:

. calculés: C: 57,13% H: 6,18% N: 9,52%

. observés: C: 57,0% H: 6,2% N: 9,5%

Exemples 3 à 10

Des bases de Schiff sont obtenues d'une façon similaire à l'exemple 1, par traitement de l'acide aspartique avec l'un quelconque des composés suivants:

. 3 - hydroxy-2-benzaldéhyde,
. 4 - hydroxy-2-chloro-5-acétophénone,
. 5 - hydroxy-2-dichloro-3,5-acétophénone,
. 6 - hydroxy-2-dichloro-3,5-benzaldéhyde,
. 7 - hydroxy-2-nitro-5-acétophénone,
. 8 - hydroxy-2-dinitro-2,5-acétophénone,
. 9 - hydroxy-2-nitro-5-benzaldéhyde,
.10 - hydroxy-2-dinitro-3,5-benzaldéhyde.

Ces bases de Schiff sont traitées successivement par de l'acide acétique glacial, puis de l'anhydride acétique, puis la L-phénylalanine méthylester.

Le mélange est hydrolysé et traité d'une façon similaire à la méthode décrite dans l'exemple 1 et l'aspartame est isolé avec de bons rendements et un fort pourcentage d'isomère α.

L'analyse élémentaire montre que les produits obtenus sont en accord avec la structure prévue.

REVENDICATIONS

1.- Procédé de production de l'aspartame par synthèse qui consiste à protéger la fonction amine libre de l'acide aspartique, à activer la fonction acide libre de l'acide aspartique protégé par production d'un anhydride interne protégé, à condenser cet anhydride interne protégé avec l'ester méthylique de phénylalanine et à déprotéger la fonction amine du dérivé obtenu pour produire l'aspartame, ce procédé étant caractérisé en ce que l'acide aspartique de départ est protégé par production d'une base de Schiff de l'acide aspartique obtenue par traitement de l'acide aspartique, d'un de ses sels alcalins ou d'un sel de cet acide avec une amine tertiaire, avec un composé oxo-aromatique de formule :

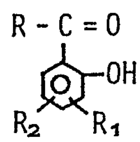

(A)

dans laquelle:

R est un atome d'hydrogène ou un radical alkyle inférieur, de préférence un radical méthyle,

$R_1$ et $R_2$, indépendamment l'un de l'autre consistent en un atome d'hydrogène ou d'halogène ou un groupe nitro ou, encore, forment ensemble un noyau aromatique.

2.- Procédé selon la revendication 1 , caractérisé en ce que la base de Schiff de l'acide aspartique est obtenue par mise en suspension dans un solvant alcoolique de l'acide aspartique et du composé oxo-aromatique (A) précité, addition d'hydroxyde de potassium (KOH) et de triéthylamine $\{N-(CH_2CH_3)_3\}$ à la suspension obtenue, addition d'acétate d'éthyle, cristallisation, filtration et séchage.

3.- Procédé selon la revendication 2, caractérisé en ce que la réaction entre l'acide aspartique et le composé oxo-aromatique est mise en oeuvre à une température comprise entre 0° et 65°C.

4.- Procédé selon une quelconque des revendications 1 à 3, caractérisé en ce que la base de Schiff ainsi produite est mise en suspension dans un agent convenable et à la suspension ainsi obtenue sont ajoutés successivement:

- un agent déshydratant;
- après au moins une heure d'agitation, l'ester méthylique de phénylalanine, en solution dans l'agent de suspension précité;

- après au moins cinq heures d'agitation, de l'acide chlorhydrique, le mélange biphasique obtenu est alors séparé et on neutralise la phase aqueuse par addition d'une solution alcaline.

5.- Procédé selon la revendication 4, caractérisé en ce que l'agent convenable pour la mise en suspension est le dichlorométhane ou l'acétate d'éthyle.

6.- Procédé selon la revendication 4 ou 5, caractérisé en ce que l'agent déshydratant consiste en un anhydride d'acide gras ou un halogénure d'acide.

7.- Procédé selon la revendication 6, caractérisé en ce que l'halogénure d'acide est le trichlorure de phosphore $PCl_3$.

8.- Procédé selon une quelconque des revendications 1 à 6, caractérisé en ce que le composé oxo-aromatique employé est l'un des composés appartenant au groupe formé par les composés suivants:

. hydroxy-2-naphtaldéhyde-1,
. hydroxy-2-benzaldéhyde,
. hydroxy-2-chloro-5-benzaldéhyde,
. hydroxy-2-nitro-5-benzaldéhyde,
. hydroxy-2-dichloro-3,5-benzaldéhyde,
. hydroxy-2-dinitro-3,5-benzaldéhyde

9.- Procédé selon l'une quelconque des revendications 1 à 6, caractérisé en ce que le composé oxo-aromatique employé est l'un des composés appartenant au groupe formé par les composés suivants :

. hydroxy-2-acétophénone,
. hydroxy-2-chloro-5-acétophénone,
. hydroxy-2- nitro -3,5-acétophénone,
. hydroxy-2-dichloro-3,5-acétophénone,
. hydroxy-2-dinitro-3,5-acétophénone

10.- Mélange d'isoméres d'aspartame enrichi en l'isomère α, obtenu à l'aide du procédé selon une quelconque des revendications 1 à 9.

Office européen des brevets

# RAPPORT DE RECHERCHE EUROPEENNE

0091330

Numéro de la demande

EP 83 40 0447

| Catégorie | DOCUMENTS CONSIDERES COMME PERTINENTS<br>Citation du document avec indication, en cas de besoin, des parties pertinentes | Revendication concernée | CLASSEMENT DE LA DEMANDE (Int. Cl. 3) |
|---|---|---|---|
| Y | AUSTRALIAN JOURNAL OF CHEMISTRY, vol. 29, no. 7, juillet 1976, pages 1591-1603, Commonwealth Scientific and Industrial Research Organization, Melbourne, AU. A.P. HOPE et al.: "The application of ketimine derivatives of amino acids to peptide synthesis" * En entier *<br>--- | 1,2 | C 07 C 103/52 |
| Y | JOURNAL OF THE AMERICAN CHEMICAL SOCIETY, vol. 84,20 juin 1962, pages 2417-2420, American Chemical Society, New York, USA J.C. SHEEHAN et al.: "The N-(2-hydroxyarylidene) protecting group in peptide synthesis" * En entier *<br>--- | 1,2 | DOMAINES TECHNIQUES RECHERCHES (Int. Cl. 3) |
| Y | FR-A-2 078 640 (BEECHAM) * En entier *<br>----- | 1,2 | C 07 C 103/00 |

Le présent rapport de recherche a été établi pour toutes les revendications

| Lieu de la recherche | Date d'achèvement de la recherche | Examinateur |
|---|---|---|
| LA HAYE | 08-07-1983 | RAJIC M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82